(19) Europäisches Patentamt
European Patent Office
Office européen des brevets

(11) EP 0 441 979 B1

(12) **EUROPEAN PATENT SPECIFICATION**

(45) Date of publication and mention of the grant of the patent:
**31.01.1996 Bulletin 1996/05**

(21) Application number: **90912956.1**

(22) Date of filing: **30.08.1990**

(51) Int. Cl.[6]: **C07C 57/30**, C07C 211/27, C07C 209/88, C07B 57/00

(86) International application number: **PCT/JP90/01107**

(87) International publication number: **WO 91/03453 (21.03.1991 Gazette 1991/07)**

# (54) OPTICALLY ACTIVE 2-(ALKYL-SUBSTITUTED PHENYL)-PROPIONIC ACID DERIVATIVE AND OPTICAL RESOLUTION OF ( )-1-METHYL-3-PHENYLPROPYLAMINE

OPTISCH AKTIVES 2-(ALKYLSUBSTITUIERTE PHENYL)-PROPIONSÄUREDERIVAT UND OPTISCHE TRENNUNG DES (+/-)-1-METHYL-3-PHENYLPROPYLAMINS

DERIVE D'ACIDE 2-(PHENYL A SUBSTITUTION ALKYLE)-PROPIONIQUE OPTIQUEMENT ACTIF ET RESOLUTION OPTIQUE DE ( )-1-METHYL-3-PHENYLPROPYLAMINE

(84) Designated Contracting States:
**DE FR GB**

(30) Priority: **07.09.1989 JP 232086/89**
**29.01.1990 JP 18747/90**

(43) Date of publication of application:
**21.08.1991 Bulletin 1991/34**

(73) Proprietor: **DAICEL CHEMICAL INDUSTRIES, LTD.**
**Sakai-shi Osaka-fu 590 (JP)**

(72) Inventors:
- **NOHIRA, Hiroyuki**
  **Saitama 338 (JP)**
- **NOHIRA, Misako**
  **Saitama 338 (JP)**
- **MIYOSHI, Haruo**
  **Kobe-shi Hyogo 673 (JP)**
- **ISHIGURO, Takeshi**
  **14-1, Shimotaya**
  **Nakakubiki-gun Niigata 944-01 (JP)**

(74) Representative: **Grünecker, Kinkeldey,**
**Stockmair & Schwanhäusser**
**Anwaltssozietät**
**D-80538 München (DE)**

(56) References cited:
**EP-A- 0 320 898**
**JP-A- 5 527 147**
**JP-A-53 149 945**

- **PATENT ABSTRACTS OF JAPAN, vol. 12, no. 37 (C-473)(2884), 4th February 1988; & JP-A-62 185 044 (SUMITOMO CHEM. CO., LTD) 13-08-1987**
- **PATENT ABSTRACTS OF JAPAN, vol. 13, no. 137 (C-582)(3485), 5th April 1989; & JP-A-63 301 844 (SUMITOMO CHEM. CO., LTD) 08-12-1988**
- **Journal of Chemical Society, Chemical Communications, No. 4, p.248-249 (1989).**
- **Journal of Organic Chemistry, Vol. 50, No. 26, p.5612-5615 (1985).**
- **Journal of Organic Chemistry, Vol. 47, No. 24, p.4692-4702 (1982).**
- **Chemistry Letters, No. 2, p.227-230 (1982).**
- **Beilsteins Handbuch Der Organischen Chemie, H. Vol. 9, p.562 (1926).**
- **Beilsteins Handbuch Der Organischen Chemie, EIII, Vol. 9, p.2563 (1971).**
- **Rec. Trav. Chim. Pays-Bas 72 (1963) 169-210**
- **JP(A) G1-126035; JP(A) 62-12740; JP(A) 61-210 04y**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 441 979 B1

## Description

The present invention relates to a novel, optically active 2-(alkylphenyl)propionic acid derivative useful as a reagent for the optical resolution of amines and as an intermediate for the preparation of various drugs, such as hypotensive drugs, and to a process for the preparation of the optically active 2-(alkylphenyl) propionic acid derivative.

1-Methyl-3-phenylpropylamine can be easily prepared as a racemic modification by ammohydrogenating 4-phenyl-2-butanone prepared by the treatment of methyl acetoacetate and benzyl chloride with an alkali. However, no proper process for preparing optically active 1-methyl-3-phenylpropylamine has been found as yet. A method of the optical resolution of a racemic modification of 1-methyl-3-phenylpropylamine characterized by treating the racemic modification with optically active mandelic acid is described in, for example, J. Vsn Dijk, V.G. Keizer and H.D. Moed, Recl. Trav. Chem. Pays-Bas, 82, 189 (1963). According to this method, however, recrystallization must be repeated several times in order to obtain a pure optically active substance and the yield is as low as 20% (described in the above literature). Thus, the above method is not always satisfactory.

Meanwhile, known optically active 2-arylpropionic acid derivatives include derivatives of 2-phenylpropionic acid, and 2-(4-isobutylphenyl)propionic, 2-(1-naphthyl)propionic, 2-(4-methoxyphenyl)propionic, 2-(2,5-dinitrophenyl)propionic and 2-(2,4-dinitrophenyl)propionic acids and derivatives thereof. Further, racemic modifications of 2-(2,5-dimethylphenyl)propionic acid and its ester are described in the Journal of Organic Chemistry, 47, 4692 (1982) and 50, 5612 (1985).

JP-A-210049/1986, JP-A-126035/1986 and JP-A-12740/1987 disclose optically active arylpropionic acids. Specifically, JP-A-126035/1986 discloses the use of 2-phenylpropionic acid for the optical resolution of amines, arylaliphatic amines, such as adrenaline and ephedrine, in particular.

It is the object of the present invention to provide a novel, optically active 2-(alkylphenyl)propionic acid derivative, a process for efficiently preparing same and its uses.

In accordance with the present invention this object is attained with an optically active 2-(alkylphenyl)propionic acid compound represented by the formula (I):

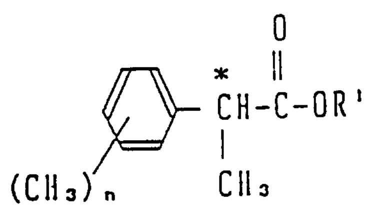

( I )

wherein n is 2 or 3; $R^1$ represents a hydrogen atom or an alkyl group having 1 to 3 carbon atoms; and C* represents an asymmetric carbon atom.

Particularly, it is preferable that the alkylphenyl group be 2,5-dimethylphenyl, 2,4-dimethyl phenyl or 2,4,6-trimethylphenyl.

Further, the present invention relates to the use of a compound of formula (I) as a reagent in optical resolution of amines and most preferably of (±)-1-methyl-3- phenylpropylamine.

Further, the present invention relates to a process for preparing a compound of formula (I) comprising the following steps:

sulfonylating L-(+) or D-(-)-lactic acid or an ester thereof,

reacting the formed sulfonyl derivative with a benzene derivative represented by the general formula (II):

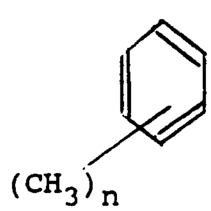

wherein n is 2 or 3, in the presence of a Lewis acid.

Particular examples of the compound represented by the general formula (I) include S and R forms of 2-(2,5-dimethylphenyl)propionic acid, 2-(2,4-dimethylphenyl)propionic acid, 2-(3,5-dimethylphenyl)propionic acid, 2-(2,3-dimethylphenyl)propionic acid, 2-(3,4-dimethylphenyl)propionic acid and 2-(2,4,6-trimethylphenyl)propionic acid; and methyl, ethyl, propyl and isopropyl esters thereof. Further, salts of these carboxylic acids with, e.g., alkali metals or alkaline earth metals also fall within the scope of the present invention.

The process for preparing a compound of formula (I) according to the present invention will now be described in details. Namely, the derivative can be easily prepared by sulfonylating L-(+) or D-(-)-lactic acid or an ester thereof and reacting the formed sulfonyl derivative with a benzene derivative represented by the general formula (II ):

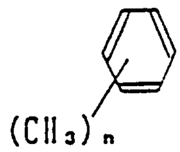

( II )

(wherein n is 2 or 3), for example, p-xylene or mesitylene, in the presence of a Lewis acid. The L-(+) or D-(-)-lactic acid to be used as a starting material may be one prepared by the optical resolution of a racemic modification of lactic acid or one prepared directly from e.g. glucose, which is available industrially at a low cost. By selecting one of the two enantiomers, the starting material can be prepared as an optically active isomer having a configuration depending upon the selected enantiomer. The reagent to be used in the above sulfonylation is preferably p-toluenesulfonyl chloride, methanesulfonyl chloride or benzenesulfonyl chloride. If necessary, this reaction may be accelerated by using a tertiary amine such as triethylamine or pyridine. Further, the reaction may be conducted in the presence of a solvent inert under the reaction conditions at a temperature of -20 to 40°C preferably -5 to 25°C. Although the reaction time varies depending upon the kinds of the reagent and tertiary amine used, the completion of the reaction can be confirmed by thin-layer chromatography. After the reaction, the reaction mixture as such is reacted with a benzene derivative represented by the formula (II ) in the presence of a Lewis acid. Alternatively, the reaction mixture may be freed from a tertiary amine salt such as triethylamine hydrochloride formed as a by-product by, e.g., filtration or washing with water prior to the reaction with e.g. p-xylene. Examples of the Lewis acid to be used therein include aluminum chloride, aluminum bromide, tin chloride, iron chloride, iron bromide, zinc chloride, boron trifluoride and titanium chloride, among which aluminum compounds such as aluminum chloride are preferable. The equivalent amount of the Lewis acid used is generally selected in the range of 0.2 to 2.4. Although a solvent inert under the reaction conditions may be used at need, the use of such a solvent can be omitted by using a compound represented by formula (II ) in an excessive amount. The reaction temperature may be from -50 to 100°C, preferably from -5 to 25°C. The reaction time is 1 to 48 h, though it varies depending upon the kind of the Lewis acid and the reaction temperature. After the completion of the reaction, the reaction mixture is poured into water and an objective compound can be isolated from the aqueous solution by e.g. extraction with an organic solvent in a high yield like in the case of an ordinary Friedel-Crafts reaction see JP-A-210049/1986. When the starting material is an ester of the lactic acid, the obtained product can be converted into a corresponding carboxylic acid by an ordinary hydrolysis in a high yield. Although the objective compound thus obtained is acceptable both optically and chemically, it may be further purified by, e.g., distillation or recrystallization.

According to the present invention (±)-1-methyl-3-phenylpropylamine can be optically resolved by treating (±)-1-methyl-3-phenylpropylamine or a salt thereof with a compound represented by the general formula (I ) or a salt thereof as a reagent for optical resolution.

The starting salt of (±)-1-methyl-3-phenylpropylamine that may be treated includes those of inorganic acids such as hydrochloric, sulfuric or phosphoric acid and those of organic acids such as p-toluenesulfonic, acetic or oxalic acid.

A preferred embodiment of the use of a compound of the present invention as a reagent for the optical resolution will now be described.

(±)-1-Methyl-3-phenylpropylamine, or a salt thereof and a compound represented by the general formula (I) or a salt thereof are added to a suitable solvent and the obtained mixture is heated to form a solution.

Preferable examples of the solvent to be used therein include water; alcohols such as methanol, ethanol, 1-propanol, 2-propanol, 1-butanol and 2-butanol; ketones such as acetone and methyl ethyl ketone; esters such as methyl acetate and ethyl acetate; aromatic hydrocarbons such as benzene, toluene and xylene; halogenated hydrocarbons such as methylene chloride, chloroform and carbon tetrachloride; and saturated aliphatic hydrocarbons such as pentane, hexane and cyclohexane. These solvents may be used alone or as a mixture prepared by mixing some of them at a suitable ratio. The amount of the compound represented by the general formula (I ) to be used is preferably 0.5 to 1.1 mol per mol of the (±)-1-methyl-3-phenylpropylamine used. The supersaturated solution prepared above gives a precipitate of a difficultly soluble diastereomeric salt by gradual cooling or the removal of the solvent by vacuum distillation. In this precipitation step, a small amount of a pure diastereomeric salt, such as (+)-1-methyl-3-phenylpropylamine (-)-2-(2,5-dimethylphenyl)propionate or (-)-1-methyl-3-phenylpropylamine (+)-2-(2,5-dimethylphenyl)propionate, is added to the supersaturated solution as a seed crystal to cause the precipitation.

Optically active (+) or (-)-1-methyl-3-phenylpropylamine can be obtained by recovering the precipitate by filtration, if necessary recrystallizing it from a solvent which may be the same as that used in the above reaction or different from it, treating it with a water-soluble base to liberate an amine, extracting the amine with an organic solvent and distilling the solvent layer. Further, the optically active carboxylic acid used as a reagent for optical resolution present as a water-soluble alkali salt can be recovered and re-used by treating the remaining aqueous solution with a mineral acid such as hydrochloric or sulfuric acid. As described above, optically active (+) or (-)-1-methyl-3-phenylpropylamine can be prepared in a high yield and at a high optical purity according to the present invention.

Each of the optically active 2-(2,5-dimethylphenyl)propionic acid and 2-(2,4,6-trimethylphenyl)propionic acid according to the present invention exhibits a higher resolution efficiency than that of optically active 2-phenylpropionic acid which has been known, when employed as a reagent for the optical resolution through the formation of diasteromeric salts. For example, when (S)-(+)-2-(2,5-dimethylphenyl)propionic acid was used in the optical resolution of (±)-1-methyl-3-phenylpropylamine, (-)-1-methyl-3-phenyl propylamine having an optical purity of 98.3% e.e. was obtained in a yield of 55.5%, while when 2-phenylpropionic acid was used as a reagent for the optical resolution, it was obtained in a yield of 69.6% and at an optical purity of at most 39.3% e.e. Further, each of the optically active 2-(2,5-dimethylphenyl)propionic acid and 2-(2,4,6-trimethylphenyl)propionic acid and derivatives thereof according to the present invention can be prepared by a simple and industrially advantageous process, which comprises sulfonylating an optically active lactic acid or ester thereof which is available at a low cost and reacting the obtained sulfonyl derivative with xylene or mesitylene in the presence of a Lewis acid, at an optically and chemically acceptable purity in a high yield. Therefore, the optical resolution with such a compound as described above according to the diastereomer method is remarkably economical. Of course, these compounds can be quantitatively recovered after the use as a reagent for optical resolution and re-used.

The present invention will now be described in more detail by referring to the following Examples.

<u>Preparation Example 1</u>

15.6 g of methyl L-lactate and 15.2 g of triethylamine were dissolved in 100 ml of methylene chloride, and 28.7 g of p-toluenesulfonyl chloride were added to the obtained solution at -10°C. The obtained mixture was stirred at room temperature for 6 h and poured into chilled water. The organic layer was separated from the aqueous layer, washed with an aqueous solution of hydrochloric acid, dehydrated, dried and freed from the solvent to give about 25 g of methyl (S)-(-)-2-p-toluenesulfonyloxyproplonate. 6.46 g of the methyl ester thus obtained were reacted with a mixture comprising 50 ml of p-xylene and 8.31 g of aluminum chloride at room temperature for 7 h. After the completion of the reaction had been confirmed by thin-layer chromatography, the reaction mixture was extracted with benzene and the benzene layer was concentrated to give 4.22 g of methyl (S)-2-(2,5-dimethylphenyl)propionate (yield: 87.8%). The characteristics of this ester were as follows:

proton nuclear magnetic resonance spectrum (ppm)
1.46 d 3H, 2.30 s 6H, 3.69 s 3H, 3.93 q 1H, 7.04 bs 3H
infrared absorption spectrum ($cm^{-1}$)
2950, 1735, 1330, 1290, 1090

2.03 g of the ester were hydrolyzed with 5 ml of concentrated hydrochloric acid in 10 ml of acetic acid at 70°C for 7.5 h. Thus, 1.59 g of (S)-2-(2,5-dimethylphenyl)propionic acid was obtained (yield: 90.2%). This acid was recrystallized from hexane. The characteristics of the product were as follows:

melting point: 103 to 104°C
specific rotation $[\alpha]_D$ = +61.6°
c = 1.6 ($CHCl_3$)
proton nuclear magnetic resonance spectrum (ppm)
1.46 d 3H, 2.30 s 6H, 3.93 q 1H, 6.09 s, 7.04 s, 11.04 bs 1H
infrared absorption spectrum ($cm^{-1}$)
1700, 1330, 960, 810

The product was analyzed with a commercially available column for optical resolution. The optical purity thereof was 99.5% e.e.

1.01 g of the above ester was hydrolyzed with 10 ml of 1.2 N NaOH at 19°C for 14 h to give 0.78 g of (S)-2-(2,5-dimethylphenyl)propionic acid (yield: 81.1%). The specific rotation, proton nuclear magnetic resonance spectrum and infrared absorption spectrum of this product were identical with those of the product prepared above by the hydrolysis with concentrated hydrochloric acid.

<u>Preparation Example 2</u>

In a similar manner to that of Preparation Example 1, methyl (R)-(+)-2-toluenesulfonyloxypropionate was prepared from methyl D-lactate and reacted with p-xylene to give methyl (R)-2-(2,5-dimethylphenyl)propionate. This ester was hydrolyzed to give (R)-2-(2,5-dimethylphenyl)propionic acid. The characteristics of the product were as follows:

melting point: 102.5 to 103.5°C
specific rotation $[\alpha]_D$ = -60.9°
c = 1.54 ($CHCl_3$)

The proton nuclear magnetic resonance spectrum and infrared absorption spectrum thereof were completely identical with those of (S)-2-(2,5-dimethylphenyl)propionic acid.

Preparation Example 3

2.29 g of the methyl (S)-(-)-2-p-toluenesulfonyloxypropionate prepared in Preparation Example 1 were reacted with a mixture comprising 20 ml of 1,3,5-trimethylbenzene (mesitylene) and 2.95 g of aluminum chloride at room temperature. After the completion of the reaction had been confirmed by thin-layer chromatography, the reaction mixture was extracted with benzene and the benzene layer was concentrated to give 1.63 g of methyl (S)-2-(2,4,6-trimethylphenyl)propionate (yield: 88.8%). The characteristics of this ester were as follows:

proton nuclear magnetic resonance spectrum (ppm)
1.42 d 3H (J=7Hz), 2.25 s 9H, 3.63 s 3H, 4.00 q 1H (J=7Hz), 6.77 bs 2H

0.58 g of the ester was hydrolyzed with 5 ml of concentrated hydrochloric acid in 10 ml of acetic acid at 70 to 80°C for 14 h to give 0.42 g of (S)-2-(2, 4,6-trimethylpheny)propionic acid. The obtained acid was recrystallized from hexane. The characteristics of the product were as follows:

melting point: 94.0 to 95.0°C
specific rotation $[\alpha]_D$ = +115.4°
c = 0.74 ($CHCl_3$)
proton nuclear magnetic resonance spectrum (ppm)
1.42 d 3H (J=7Hz), 2.25 s 9H, 4.07 q 1H (J=7Hz), 6.77 bs 2H, 10.44 bs 1H

Resolution Example 1

3.74 g (25 mmol) of (±)-1-methyl-3-phenylpropylamine [hereinafter abbreviated to "(±)-MPPA") and 3.56 g (20 mmol) of (+)-2-(2,5-dimethylphenyl)propionic acid [hereinafter abbreviated to "(+)-DMP"] were added to 20 ml of 2-butanone, and the obtained mixture was heated to give a solution. This solution was cooled to room temperature and allowed to stand overnight to precipitate a crystal. This crystal was recovered by filtration to give 3.15 g (9.63 mmol) of crude (-)-MPPA.(+)-DMP salt. This salt was recrystallized twice from 25 ml of 2-butanone and from 15 ml thereof successively to give 2.20 g (6.74 mmol) of pure (-)-MPPA.(+)-DMP salt. M.p.: 111 to 111.5°C, and $[\alpha]^{25}_{435}$ -13.9° (c=1.1, MeOH). The yield was 53.% based on the (-)-MPPA contained in the (±)-MPPA used. 4.2 ml of a 2N aqueous solution of sodium hydroxide were added to the pure (-)-MPPA·(+)-DMP salt and the obtained mixture was extracted with cyclohexane. The organic layer was dried over granular potassium hydroxide and freed from the solvent by vacuum distillation to give 1.00 g (6.71 mmol) of (-)-MPPA. This product exhibited a specific rotation, $[\alpha]_{589}$, of -17.4° (c=3, cyclohexanone). Further, the product was acetylated by a conventional process and analyzed with a commercially available column for optical resolution. The optical purity thereof was 94.3% e.e. On the other hand, the (+)-DMP used as a reagent for optical resolution was recovered by acidifying the aqueous layer with concentrated hydrochloric acid.

Resolution Example 2

3.74 g (25 mmol) of (±)-MPPA and 3.56 g (20 mmol) of (+)-DMP were added to 7.5 ml of 2-butanone, and the obtained mixture was heated to form a solution. This solution was cooled to room temperature and allowed to stand overnight to precipitate a crystal. This crystal was recovered by filtration to give 3.94 g (12.0 mmol) of crude (-)-MPPA·(+)-DMP salt. This salt was recrystallized from 8 ml of 2-butanone four times to give 2.27 g (6.94 mmol) of pure (-)-MPPA·(+)-DMP salt. M.p.: 113 to 114°C, and $[\alpha]^{23}_{435}$ : -13.9° (c=1, MeOH), The yield was 55.5% based on the (-)-MPPA contained in the (±)-MPPA used.1,2 ml of a 1N aqueous solution of sodium hydroxide was added to 329 mg of the salt and the obtained mixture was extracted with 5 ml of cyclohexanone. The organic layer was dried over granular potassium hydroxide. The obtained product exhibited a specific rotation, $[\alpha]_{589}$, of -18.2° (c=3, cyclohexanone). Further, it exhibited an optical purity of 98.3% e.e. as analyzed in a similar manner to that of Resolution Example 1.

Resolution Example 3

0.757 g (5.1 mmol) of (±)-MPPA and 0.742 g (4.2 mmol) of (+)-DMP were added to a mixture comprising 3 ml of methanol and 3 ml of water, and the obtained mixture was heated to form a solution. This solution was cooled to room temperature. A seed crystal of (-)-MPPA·(+)-DMP salt was added to the solution and the resulting solution was allowed to stand overnight to precipitate a crystal. This crystal was recovered by filtration to give 492 mg (1.50 mmol) of crude (-)-MPPA·(+)-DMP salt. 468 mg of this salt were dissolved in 2.5 ml of methanol, followed by the dropping of 2.5 ml of

water thereinto. The obtained mixture was stirred to precipitate a crystal. This crystal was recovered by filtration to give 321 mg (0.98 mmol) of pure (-)-MPPA (+)-DMP salt. M.p.: 115 to 115.5°C, and $[\alpha]^{25}_{435}$ : -15.0° (c=1, MeOH). 1.3 ml of a 1N aqueous solution of sodium hydroxide was added to the salt and the obtained mixture was extracted with 5 ml of cyclohexanone. The organic layer was dried over granular potassium hydroxide. The obtained product exhibited a specific rotation, $[\alpha]^{29}_{589}$ , of -17.1° (c=3, cyclohexanone). Further, it exhibited an optical purity of 92.4% e.e. as analyzed in a similar manner to that of Resolution Example 1.

Comparative Resolution Example

0.74 g (5 mmol) of (±)-MPPA and 0.73 g (4.9 mmol) of (-)-2-phenylpropionic acid (hereinafter abbreviated to "(-)-PPA") were added to 2 ml of 2-butanone and the obtained mixture was heated to form a solution. This solution was cooled to room temperature and allowed to stand overnight. The precipitated crystal was recovered by filtration to give 0.71 g (2.37 mmol) of crude (+)-MPPA·(-)-PPA salt. This salt was recrystallized from 2 ml of 2-butanone to give 0.52 g (1.74 mmol) of pure (+)-MPPA (-)-PPA salt. M.p.: 104 to 105°C, and $[\alpha]^{28}_{435}$ : -10.7° (c=1, MeOH). The yield was 69.6% based on the (+)-MPPA contained in the (±)-MPPA used. 2 ml of a 1N aqueous solution of sodium hydroxide was added to 0.47 g of the salt and the obtained mixture was extracted with 3.5 ml of cyclohexane. The organic phase was dried over granular potassium hydroxide. The obtained product exhibited a specific rotation, $[\alpha]^{29}_{589}$ , of +7.27° (c=4, cyclohexane). Further, it exhibited an optical purity of 39.3% e.e. as analyzed in a similar manner to that of Resolution Example 4.

Preparation Example 4

8.60 g of methyl (S)-(-)-2-p-toluenesulfonyloxypropionate prepared from methyl L-lactate in a similar manner to that of Preparation Example 1 was added to a mixture comprising 20 ml of m-xylene and 8.78 g of aluminum chloride and the obtained mixture was reacted at room temperature for 20 h. After the completion of the reaction had been confirmed by thin-layer chromatography, the reaction mixture was poured into a mixture comprising 12 ml of concentrated hydrochloric acid and 33 ml of water. The organic layer was washed with water, concentrated and distilled to give 4.53 g of a mixture comprising methyl S-2-(2,4-dimethylphenyl)propionate and methyl S-2-(3,5-dimethylphenyl)propionate. 4.06 g of this ester mixture was hydrolyzed with 20 ml of 1.2N sodium hydroxide at 25°C for 18 h. The reaction mixture was acidified by the addition of 5 ml of 6N hydrochloric acid and extracted with 50 ml of chloroform. The chloroform layer was distilled to give 3.09 g of a mixture comprising S-2-(2,4- dimethylphenyl)propionic acid and S-2-(3,5- dimethylphenyl)propionic acid.

100 mg of this mixture was separated by liquid chromatography into 75 mg of S-2-(2,4-dimethylphenyl)propionic acid and 14 mg of S-2-(3,5-dimethylphenyl)propionic acid. The characteristics of these compounds were as follows:

S-2-(2,4-dimethylphenyl)propionic acid
- specific rotation $[\alpha]^{21.0}_{D}$ = +83.75° (c=1.06, $CHCl_3$)
- proton nuclear magnetic resonance spectrum (ppm)
- 1.47 d 3H (J=7Hz), 2.28 s 3H, 2.33 s 3H, 3.93 q 1H (J=7Hz)

S-2-(3,5-dimethylphenyl)propionic acid
- proton nuclear magnetic resonance spectrum (ppm)
- 1.48 d 3H (J=7Hz), 2.30 s 6H, 3.65 q 1H (J=7Hz)

Resolution Example 4

1.49 g (10 mmol) of (±)-MPPA was dissolved in 10 ml of a 1N aqueous solution of hydrochloric acid to give a solution. Another solution of 0.89 g (5 mmol) of (-)-DMP in 5 ml of a 1N aqueous solution of sodium hydroxide was added to the above solution under mild stirring to precipitate a crystal. This crystal was recovered by filtration to give 1.35 g (4.13 mol) of crude (+)-MPPA (-)-DMP salt (m.p.: 110.5 to 114.5°C, $[\alpha]^{26.0}_{435}$ : +19.8° (c=1.0, MeOH)). The yield was 82.6% based on the (+)-MPPA contained in the (±)-MPPA used. 0.95 g (2.91 mmol) of the salt was dissolved in 2.9 ml of a 1N aqueous solution of sodium hydroxide to give a solution. 2.9 ml of 1N hydrochloric acid was added to this solution under stirring to precipitate a crystal. This crystal was recovered by filtration to give 0.81 g (2.66 mmol) of pure (+)-MPPA·(-)-DMP salt. M.p.: 115.5 to 117.5°C, and $[\alpha]^{25}_{435}$ : +18.3° (c=1, MeOH). The yield was 70.3% based on the (+)-MPPA contained in the (±)-MPPA used. 1.2 ml of 1 N sodium hydroxide was added to 330 mg of this salt and the obtained mixture was extracted with 5 ml of cyclohexane. The organic layer was dried over granular potassium hydroxide. The product exhibited a specific rotation, $[\alpha]^{31}_{D}$ , of +16.4 (c=3, cyclohexane). Further, it exhibited an optical purity of 94.6% e.e. as analyzed in a similar manner to that of Example 4.

Resolution Example 5

187 mg (1.25 mmol) of (±)-MPPA and 178 mg (1.0 mmol) of (+)-2-(2,4-dimethylphenyl)propionic acid [hereinafter abbreviated to "(+)-2,4-DMP"] were dissolved in 2 ml of 2-butanone and the obtained solution was allowed to stand at 4°C overnight. The precipitated crystal was recovered by filtration to give 212 mg (0.65 mmol) of (-)-MPPA·(-)-2,4-DMP salt. The yield was 103.7% based on the (-)-MPPA contained in the (±)-MPPA used. An aqueous solution of sodium hydroxide was added to the salt and the obtained mixture was extracted with benzene. The organic layer was acetylated and analyzed with a commercially available column for optical resolution. The optical purity thereof was 68.7% e.e.

## Claims

1. An optically active 2-(alkylphenyl)- propionic acid compound represented by the formula (I):

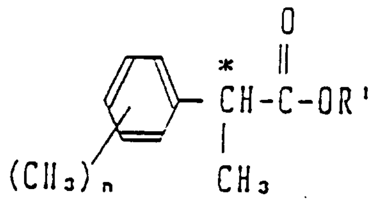

( I )

wherein n is 2 or 3; $R^1$ represents a hydrogen atom or an alkyl group having 1 to 3 carbon atoms; and C* represents an asymmetric carbon atom.

2. The compound according to claim 1, wherein the alkylphenyl group is 2,5-dimethylphenyl, 2,4-dimethylphenyl or 2,4,6-trimethylphenyl.

3. A process for preparing a compound according to claim 1 or 2 comprising the following steps:
sulfonylating L-(+) or D-(-)-lactic acid or an ester thereof,
reacting the formed sulfonyl derivative with a benzene derivative represented by the general formula (II):

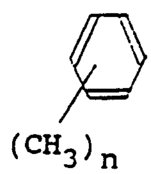

wherein n is 2 or 3, in the presence of a Lewis acid.

4. The use of a compound according to claim 1 or 2 for the optical resolution of amines.

5. The use of claim 4 wherein the amine is (±)-1-methyl-3-phenylpropylamine.

6. The use of a compound according to claim 1 or 2 as an intermediate for the preparation of various drugs.

**Patentansprüche**

1. Optisch aktive 2-(Alkylphenyl)propionsäure-Verbindung, dargestellt durch die Formel (I):

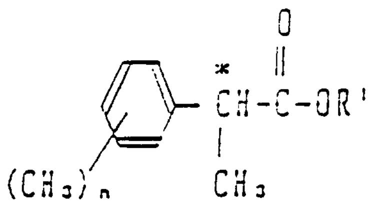

(I)

worin n 2 oder 3 ist; $R^1$ bedeutet ein Wasserstoffatom oder eine Alkylgruppe mit 1 bis 3 Kohlenstoffatomen; und C* bedeutet ein asymmetrisches Kohlenstoffatom.

2. Verbindung nach Anspruch 1, worin die Alkylphenylgruppe eine 2,5-Dimethylphenyl-, 2,4-Dimethylphenyl- oder 2,4,6-Trimethylphenylgruppe ist.

3. Verfahren zur Herstellung einer Verbindung nach Anspruch 1 oder 2, umfassend die folgenden Schritte:
Sulfonylieren von L-(+)- oder D-(-)-Milchsäure oder eines Esters davon,
Umsetzen des gebildeten Sulfonylderivats mit einem Benzolderivat, dargestellt durch die allgemeine Formel (II):

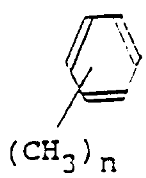

worin n 2 oder 3 ist, in Gegenwart einer Lewis-Säure.

4. Verwendung einer Verbindung nach Anspruch 1 oder 2 für die Antipodentrennung von Aminen.

5. Verwendung nach Anspruch 4, worin das Amin (±)-1-Methyl-3-phenylpropylamin ist.

6. Verwendung einer Verbindung nach Anspruch 1 oder 2 als Zwischenprodukt für die Herstellung von verschiedenen Arzneimitteln.

**Revendications**

1. Composé de type acide 2-(alkylphényl)propionique optiquement actif, représenté par la formule (I):

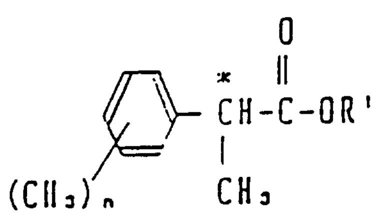

(I)

dans laquelle n est 2 ou 3; $R^1$ représente un atome d'hydrogène ou un groupe alkyle ayant de 1 à 3 atomes de carbone; et C* représente un atome de carbone asymétrique.

2. Composé selon la revendication 1, dans lequel le groupe alkylphényle est le groupe 2,5-diméthylphényle, 2,4-diméthylphényle ou 2,4,6-triméthylphényle.

3. Procédé pour la préparation d'un composé selon la revendication 1 ou 2, comprenant les étapes suivantes:
sulfonation de l'acide L-(+)- ou D-(-)-lactique ou d'un ester de celui-ci,
mise en réaction du dérivé sulfonyle obtenu, avec un dérivé du benzène représenté par la formule générale (II):

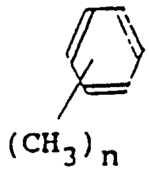

dans laquelle n est 2 ou 3, en présence d'un acide de Lewis.

4. Utilisation d'un composé selon la revendication 1 ou 2, pour la résolution optique d'amines.

5. Utilisation selon la revendication 4, dans laquelle l'amine est la (±)-1-méthyl-3-phénylpropylamine.

6. Utilisation d'un composé selon la revendication 1 ou 2, en tant que produit intermédiaire pour la fabrication de divers médicaments.